# EUROPEAN PATENT APPLICATION

(11) **EP 4 563 588 A1**
(43) Date of publication of application: **04.06.2025**
(21) Application number: 23846865.6
(22) Date of filing: 10.07.2023
(51) Int. Cl.: C07K 14/00, A01N 63/50, A61K 38/00, A61P 31/04, C12N 15/70, C12R 1/19

(54) **COMPOSITION OF ANTIBACTERIAL PROTEIN FOR TREATING BOVINE MASTITIS**

(30) Priority: 26.07.2022 KR 20220092200
(71) Applicant: Intron Biotechnology, Inc., Seongnam-si, Gyeonggi-do 13202 (KR)
(72) Inventor: YOON, Seong Jun, Seoul 06281 (KR); JUN, Soo Youn, Seoul 06518 (KR); JUNG, Gi Mo, Seoul 08725 (KR); CHOI, Ji Hye, Seongnam-si Gyeonggi-do 13245 (KR); KANG, Sang Hyeon, Seoul 05501 (KR)
(74) Representative: Latscha Schöllhorn Partner AG
(86) International application number: PCT/KR2023/009725
(87) International publication number: WO 2024/025206

(57) **Abstract**

The present invention relates to an antibacterial protein SSL200 having a specific antibacterial activity against Staphylococci including *Staphylococcus aureus.* More specifically, the present invention relates to: an antibacterial protein SSL200 specific for Staphylococci including Staphylococcus aureus, the protein being characterized by having the ability to specifically lyse Staphylococci including *Staphylococcus aureus* and having an amino acid sequence represented by SEQ ID NO: 1; and a composition for treating infections caused by Staphylococci including *Staphylococcus aureus,* the composition comprising, as an active ingredient, the antibacterial protein SSL200 specific for Staphylococci including *Staphylococcus aureus.*

## Description

### Technical Field

The present disclosure relates to an antibacterial protein SSL200 and a composition containing the antibacterial protein SSL200 as an active ingredient, the antibacterial protein SSL200 having lytic activity against *Staphylococci,* including *Staphylococcus aureus,* which is one of the causative bacteria of bovine mastitis. More specifically, the present disclosure relates to the antibacterial protein SSL200 specific for *Staphylococci,* including *Staphylococcus aureus,* in which the antibacterial protein SSL200 has an ability to specifically lyse *Staphylococci,* including *Staphylococcus aureus,* and contains an amino acid sequence represented by SEQ ID NO: 1, and the present disclosure relates to the composition for treating bovine mastitis, containing the antibacterial protein SSL200, specific for *Staphylococci,* including *Staphylococcus aureus,* as an active ingredient.

### Background Art

Bovine mastitis is a disease in which bacteria, which are widely distributed in nature, invade the dairy cow's udder and cause inflammation. Bovine mastitis is the most common disease among dairy cows. Bovine mastitis increases the number of bacteria and somatic cells in milk, resulting in a decrease in milk quality. Bovine mastitis is also a representative dairy cow disease that causes economic losses such as reduced milk flow and treatment costs. Even in the developed country of the United States, the economic loss caused by bovine mastitis is enormous. It is known to cause economic losses of $200 per dairy cow, or $200 million per year.

Bovine mastitis is caused by very complex factors. Factors causing bovine mastitis may be broadly divided into three categories: microorganisms, which are causative bacteria; dairy cows, which are hosts; and environmental factors that affect them all. There are more than 100 types of bacteria known to cause bovine mastitis, and the bacteria are known to cause infections through a wide variety of routes and show diverse disease patterns.

The isolation rates of major causative agents of bovine mastitis have been reported as approximately 26% for *Staphylococcus aureus,* 26% for *Coagulase-negative staphylococci* (CNS), 6% for *Streptococci,* 6% for *Enterococci,* and 36% for gram-negative bacteria, including *Escherichia coli.* However, the isolation rates for these causative bacteria vary slightly from report to report. Although the causative bacteria of bovine mastitis are diverse, the most difficult to eradicate is bovine mastitis caused by *Staphylococcus aureus.* Therefore, the development of an effective drug for treating bovine mastitis caused by *Staphylococcus aureus* is of utmost importance and is urgently needed.

Treatments for bovine mastitis include ointments containing antibiotics such as cephalosporin-based antibiotics and injections containing herbal ingredients such as phytolacca decandra extract. There is great dissatisfaction with the usefulness of these treatments due to insufficient treatment effectiveness and frequent occurrence of resistant bacteria. Considering this, there is a need to develop treatment agents for bovine mastitis utilizing new drugs that provide improved treatment effectiveness and lower incidence of resistance.

### Disclosure

### Technical Problem

Accordingly, as a solution to the problems caused by the use of the conventional antibiotics against the harmful pathogenic bacteria *Staphylococcus aureus,* the present inventor(s) seek to provide an antibacterial protein SSL200 which can specifically lyse *Staphylococci,* including *Staphylococcus aureus.* Furthermore, the present inventor(s) further seek to provide a composition which includes this antibacterial protein as an active ingredient and can be used to treat infections caused by *Staphylococci,* including *Staphylococcus aureus.* Furthermore, the present inventor(s) further seek to provide a method of effectively treating bovine mastitis caused by *Staphylococcus aureus* using the composition.

Therefore, one objective of the present disclosure is to provide an antibacterial protein SSL200 having an antibacterial activity to specifically lyse *Staphylococci,* including *Staphylococcus aureus,* and containing an amino acid sequence represented by SEQ ID NO: 1.

Another objective of the present disclosure is to provide a method of efficiently preparing an antibacterial protein SSL200 having an antibacterial activity to specifically lyse *Staphylococci,* including the *Staphylococcus aureus* and containing an amino acid sequence represented by SEQ ID NO: 1. In this regard, *Escherichia coli* BM-2 is provided as a production strain of the antibacterial protein SSL200. The production strain *Escherichia coli* BM-2 was deposited at the Biological Resource Center of the Korea Research Institute of Bioscience and Biotechnology (KRIBB) on July 12, 2022 (Accession No. KCTC15030BP).

A further objective of the present disclosure is to provide a composition for treating bovine mastitis caused by *Staphylococcus aureus,* containing the antibacterial protein SSL200 capable of specifically lysing *Staphylococci,* including *Staphylococcus aureus,* as an active ingredient.

In addition, a yet further objective of the present disclosure is to provide a method of treating bovine mastitis caused by *Staphylococcus aureus,* by using the composition containing the antibacterial protein SSL200 as an active ingredient.

### Technical Solution

To achieve the objectives, the inventor(s) of the present disclosure utilized information on various antibacterial proteins known to have antibacterial activity against various bacterial species, and utilized their knowledge and expertise to the fullest to prepare various antibacterial protein candidates in the form of recombinant proteins and examined lytic activity of the antibacterial protein candidates against *Staphylococci,* including *Staphylococcus aureus.* Thereby, the inventor(s) selected an antibacterial protein with excellent lytic activity and developed a method of efficiently preparing the antibacterial protein, and lastly developed a composition that can be used for treating bovine mastitis caused by *Staphylococcus aureus* by using the antibacterial protein as an active ingredient. As a result, the inventor(s) completed the present disclosure.

Therefore, according to one aspect of the present disclosure, the present disclosure provides an amino acid sequence of an antibacterial protein SSL200, which may specifically lyse *Staphylococci,* including *Staphylococcus aureus.* Specifically, the amino acid sequence of the antibacterial protein SSL200 corresponds to an amino acid sequence represented by SEQ ID NO: 1. The antibacterial protein SSL200, which may specifically lyse *Staphylococci,* including *Staphylococcus aureus* is made of 325 amino acid residues and has a molecular weight of approximately 35.9 kDa.

It is obvious that the amino acid sequence represented by SEQ ID NO: 1 may be partially modified by those skilled in the art using known techniques. The modifications include partial substitution of the amino acid sequence, partial addition of the amino acid sequence, and partial deletion of the amino acid sequence. However, it is most preferable to apply the amino acid sequence represented by SEQ ID NO: 1 disclosed in the present disclosure.

Additionally, the present disclosure provides a production strain *Escherichia coli* BM-2, which can be used to produce the antibacterial protein SSL200 containing the amino acid sequence represented by SEQ ID NO: 1. The production strain *Escherichia coli* BM-2 is a production strain to produce the antibacterial protein SSL200, constructed by the present inventor(s) by transforming *E. coli* with a plasmid, containing a nucleic acid sequence represented by SEQ ID NO: 2 (5,045 bp).

According to another aspect of the present disclosure, the present disclosure provides a composition containing the antibacterial protein SSL200 as an active ingredient for being effectively used for treating infections caused by *Staphylococci,* including *Staphylococcus aureus,* in which the antibacterial protein SSL200 contains the amino acid sequence represented by SEQ ID NO: 1 and has specific lytic activity against *Staphylococci,* including *Staphylococcus aureus.*

According to the present disclosure, the antibacterial protein SSL200, which is included in the composition of the present disclosure, contains the amino acid sequence represented by SEQ ID NO: 1 according to the present disclosure, and has specific lytic activity against *Staphylococci,* including *Staphylococcus aureus,* as described above, specifically lyses *Staphylococci,* including *Staphylococcus aureus.* Thus, the antibacterial protein SSL200 is effective in treating various diseases caused by *Staphylococci,* including *Staphylococcus aureus.* Thus, the composition of the present disclosure is developed as an antibiotic, a disinfectant, a sterilizer, and a therapeutic agent to treat various diseases caused by *Staphylococci,* including *Staphylococcus aureus.*

Therefore, according to a further aspect of the present disclosure, the present disclosure provides a method of treating various diseases caused by *Staphylococci,* including *Staphylococcus aureus,* by administering the antibacterial protein SSL200 to individuals infected with *Staphylococci,* including *Staphylococcus aureus,* the antibacterial protein S2200 containing the amino acid sequence represented by SEQ ID NO: 1 and being specific for *Staphylococci,* including *Staphylococcus aureus.*

The term "disease caused by *Staphylococci",* as used herein, refers to a disease caused by infections caused by *Staphylococci,* including *Staphylococcus aureus.* The disease refers to skin diseases, bacteremia, sepsis, endocarditis, or ulcers, but is not limited thereto.

It does not matter, in this specification, whether *Staphylococci,* including *Staphylococcus aureus* is sensitive to conventional antibiotics or resistant to conventional antibiotics. In other words, it does not matter whether *Staphylococci* acquires resistance to conventional antibiotics.

The term "treatment" or "treatment", as used herein, refers to all actions which suppress infections caused by *Staphylococci,* including *Staphylococcus aureus,* and alleviate pathological conditions of diseases caused by *Staphylococci,* including *Staphylococcus aureus.*

To increase efficiency for this purpose, antibacterial substances, which can provide antibacterial activity against other bacterial species, can be added to the composition of the present disclosure.

A pharmaceutically acceptable carrier included in the composition of the present disclosure is one commonly used in preparation. The pharmaceutically acceptable carrier may include any one selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but is not limited thereto. In addition to the ingredients, the composition of the present disclosure may further include any one selected from the group consisting of lubricants, wetting agents, sweeteners, flavoring agents, emulsifiers, suspending agents, and preservatives.

The composition of the present disclosure may be administered through oral administration or parenteral administration. In the case of parenteral administration, the composition may be administered using intravenous administration, intraperitoneal administration, intramuscular administration, subcutaneous administration, or local administration. In addition, application or spraying to the affected area may also be used, but the administration method is not limited thereto.

The composition of the present disclosure may be formulated using the pharmaceutically acceptable carrier and/or an excipient by a method which can be easily performed by those skilled in the art to which the present disclosure pertains. Thereby, the composition of the present disclosure may be prepared in unit dosage form or may be prepared by placing the pharmaceutical composition in a multi-dose container. In this case, the formulation of the pharmaceutical composition may be in the form of a solution, suspension, or emulsion in an oil or aqueous medium or may be in the form of an extract, a powder, a granule, a tablet, or a capsule. In the formulation, a dispersant or stabilizer may be further included.

In addition, suitable application, spraying, and dosage of the composition vary depending on factors such as formulation method, administration method, age, weight, sex, severity of disease symptoms, food, administration time, administration route, excretion rate, and reaction sensitivity. Usually, a skilled doctor or veterinarian can easily determine and prescribe an effective dosage for the desired treatment.

The composition of the present disclosure may be developed as an antibiotic, a disinfectant, a sterilizer, and a therapeutic agent.

### Advantageous Effects

A method of treating infections caused by *Staphylococci,* including *Staphylococcus aureus,* by using a composition containing an antibacterial protein SSL200 as an active ingredient, in which the antibacterial protein SSL200 contains an amino acid sequence represented by SEQ ID NO: 1 according to the present disclosure, can also be effective against *staphylococci* which have acquired resistance to conventional antibiotics or antibacterial substances. On the other hand, the antibacterial protein SSL200 of the present disclosure specific for *Staphylococci,* including *Staphylococcus aureus* does not affect normal flora other than *Staphylococci,* including *Staphylococcus aureus,* so it can minimize side effects resulting from the use of the composition containing the antibacterial protein SSL200 as an active ingredient. Meanwhile, conventional antibiotics have the disadvantages of harming beneficial bacteria and causing various side effects.

### Description of Drawings

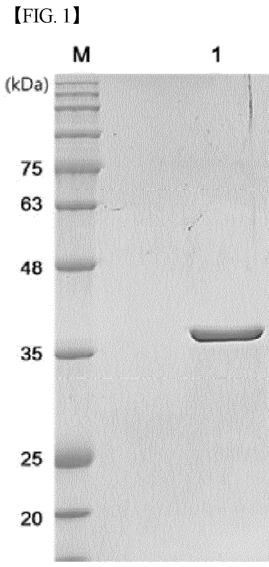

FIG. 1 shows an electrophoresis photograph showing the results of preparing an antibacterial protein SSL200 specific for *Staphylococci,* including *Staphylococcus aureus* in the form of a recombinant protein, the antibacterial protein SSL200 containing an amino acid sequence represented by SEQ ID NO: 1, in which lane M represents a protein size marker, and lane 1 represents a chromatography flow through during secondary purification.

### Best Mode

Hereinafter, the present disclosure will be described in more detail based on examples, but these examples are only illustrative of the present disclosure, and the scope of the present disclosure is not limited to these examples.

### Example 1: Preparation of Antibacterial Protein SSL200 Specific for Staphylococci, Including Staphylococcus aureus

Preparation of an antibacterial protein SSL200 specific for *Staphylococci,* including *Staphylococcus aureus,* will be described below. In this example, *Escherichia coli* BM-2 was used as a production strain, constructed by the present inventor(s) by transforming *E. coli* with the use of plasmid containing a nucleic acid sequence represented by SEQ ID NO. 2.

20 µL of *Escherichia coli* BM-2 was inoculated into 20 mL of LB medium (Tryptone 10 g/L, yeast extract 5 g/L, and NaCl 10 g/L) supplemented with 50 µg/mL kanamycin. Afterward, the LB medium was cultured with shaking at a temperature of 37°C overnight. The next day, the overnight cultured medium was added at a volume ratio of 1/100 to 1 L of LB medium supplemented with 50 µg/mL kanamycin. Culture was performed at a temperature of 37°C with a stirring speed of 200 rpm. When the cell concentration reached 0.3 to 0.35 based on absorbance at a wavelength of 600 nm, the culture temperature was changed to 19°C. After about 30 minutes, when the cell concentration reached 0.5 based on absorbance at a wavelength of 600 nm, L-arabinose was added so that the final concentration was 0.2%. Thereby, expression of an antibacterial protein SSL200 containing an amino acid sequence represented by SEQ ID NO: 1 was induced. Thereafter, an additional 16-hour culture was performed.

After completion of the culture, the cell culture broth was taken and centrifuged at a temperature of 4°C for 10 minutes at 6,000 rpm to recover the cell pellet. The recovered cell pellet was suspended in 40 mL of a buffer (50 mM Na₂HPO₄, 10 mM EDTA, 1 mM DTT, and pH 7.5). The cells of the cell suspension prepared in this way were disrupted using sonication. The sonication was performed by applying ultrasonic waves for 3 seconds to break the cells, stopping for 3 seconds. This process was repeated for a total of 15 minutes and in an ice bath.

After the cell disruption, the cell lyste was centrifuged at 13,000 rpm for 20 minutes at a temperature of 4°C, and the obtained supernatant was subjected to conventional cation-exchange chromatography and hydrophobic interaction chromatography purification processes.

The purification process is briefly explained as follows. In this example, 5 mL of HiTrap ^{™}SP FF (GE Healthcare) was used as a cation-exchange resin, and 5 mL of Toyopearl PPG-600M (Tosoh Bioscience) was used as a hydrophobic interaction resin. In a first purification process, cation-exchange chromatography was performed after pre-equilibrating a column with a buffer A (25 mM Na₂HPO₄, 10 mM EDTA, pH 7.5). A sample was loaded onto the column. Thereafter, the column was washed at a flow rate of 5 mL/min under the following conditions: 1.5 column volumes (CV) of the buffer A, 30 CV of a buffer B (25 mM Na₂HPO₄, 10 mM EDTA, 50 mM NaCl, 0.5% Triton X-100, and pH 7.5), and a mixture of the buffer A to a buffer C (25 mM Na₂HPO₄, 10 mM EDTA, 0.7 M NaCl, and pH 7.5), in which the mixture was adjusted so that the buffer C constituted 31% of the total volume. After the washing, cation-exchange chromatography, which was the first purification process, was performed under the following condition that a concentration gradient from the buffer A to the buffer C was changed from 31% to 100% at a flow rate of 5 mL/min. Next, hydrophobic interaction chromatography, which was a second purification process, was performed using the obtained first purification eluate. The hydrophobic interaction chromatography was performed after pre-equilibrating a column with a buffer D (25 mM Na₂HPO₄, 1.5 M NaCl, and pH 7.5). The sample obtained during the first purification process was added dropwise to the column using 4 M NaCl to have the same conductivity value as the buffer D. Next, the column was washed by flowing 10 CV of a buffer E (10 mM L-Histidine, 1.5 M NaCl, and pH 7.5) at a flow rate of 5 mL/min. After the washing, chromatography was performed under the following condition that a concentration gradient from the buffer E to a buffer F (10 mM L-Histidine, 1.0 M Urea, and pH 7.5) was changed from 0% to 100% at a flow rate of 5 mL/min. In this process, elution of the antibacterial protein SSL200 containing the desired amino acid sequence represented by SEQ ID NO: 1 was achieved. FIG. 1 shows the results of electrophoresis analysis of the antibacterial protein SSL200 purified using a chromatographic purification process.

Among the obtained purified fractions, those containing high concentrations of the antibacterial protein SSL200 were collected. The collected fractions were dialyzed against a buffer (10 mM L-Histidine, and pH 6.0) to perform medium exchange. Through this, it was possible to secure the antibacterial protein SSL200 solution with a purity of 90% or more.

Buffer exchange was performed for this concentrated SSL200 solution with a buffer (10 mM L-Histidine, 5%(w/v) Sorbitol, 0.1%(w/v) poloxamer 188, and pH 6.0). Thereby, "an SSL200 protein solution" was prepared.

### Example 2: Preparation of Pharmaceutical Composition for Bovine Mastitis Treatment

Using the purified SSL200 solution, an ointment formulation with a composition of "allantoin 2 g/L, sodium benzoate 0.6 g/L, sodium proionate 3 g/L, propylen glycol 50 g/L, SSL200 protein solution 3.6 ml/L" was prepared.

Specifically, the ointment formulation was prepared in the following manner. First, 90 L of sterilized distilled water was added to a preparation tank and then cooled to 30°C or less. Then, the following ingredients-allantoin, sodium propionate, sodium benzoate, and propylene glycol-were sequentially added and dissolved under mixing conditions of 170 rpm. Next, the mixing rpm of the preparation tank was lowered to 100 rpm or less. Then, the SSL200 protein solution prepared in Example 1 was slowly added. Finally, the amount of the mixture was adjusted to a final amount using distilled water, and then nitrogen gas injection was stopped. Afterward, the final amount was adjusted using purified water. In this way, the preparation of the bovine mastitis treatment was completed and the prepared product was refrigerated.

### Example 3: Investigation of Antibacterial Activity of Antibacterial Protein SSL200

Antibacterial activity of the antibacterial protein SSL200 specific for *Staphylococci,* including *Staphylococcus aureus,* and prepared according to Example 1 was examined. Details of test strains subject to antibacterial activity investigation are shown in Table 1.

**[Table 1]**

| Test Strains Subject to Antibacterial Activity Investigation | | |
|---|---|---|
| Species | Strain | Source |
| *Staphylococcus aureus* | ATCC 33591 | ATCC |
| | ATCC 35556 | |
| | CCARM 3516 | CCARM |
| | CCARM 3795 | |
| *Staphylococcus epidermidis* | KCTC 3752 | KCTC |
| *Staphylococcus haemolyticus* | ATCC 29970 | ATCC |
| *Acinetobacter baumannii* | CCARM 12226 | CCARM |
| *Clostridioides difficile* | CCARM 0185 | CCARM |
| *Pseudomonas aeruginosa* | CCARM 2252 | CCARM |
| *Klebsiella pneumoniae* | CCARM 10263 | CCARM |
| *Escherichia coli* | CCARM 1G937 | CCARM |
| ATCC: American Type Culture Collection; | | |
| CCARM: Culture Collection of Antimicrobial Resistance Microbes; | | |
| KCTC: Korean Collection for Type Cultures | | |

A turbidity reduction assay and MIC (Minimum Inhibitory Concentration) test were used to investigate the antibacterial activity. The turbidity reduction assay was conducted as follows. Test strains were suspended in physiological saline so that absorbance of the prepared cell suspension was about 0.7 at a wavelength of 600 nm. Thereafter, 0.1 mL of SSL200 solution was added (final concentration: 2.5 µM) to 0.9 mL of this cell suspension. Then, the absorbance was measured at a wavelength of 600 nm for 30 minutes. For preparing a negative control, a buffer (10 mM L-Histidine, 5% (w/v) Sorbitol, 0.1% (w/v) poloxamer 188, and pH 6.0) without the SSL200 protein was used instead of the SSL200 protein solution. An MIC was determined by conducting a routine MIC test in accordance with CLSI guidelines.

As a result, the antibacterial protein SSL200 showed lytic activity only against *Staphylococci,* including *Staphylococcus aureus,* meanwhile, the antibacterial protein S2200 did not show lytic activity against other test strains. The results are presented in Table 2.

**[Table 2]**

| Antibacterial Activity Investigation Results | | | |
|---|---|---|---|
| Species | Strain | Susceptibility | MIC (µg/ml) |
| *Staphylococcus aureus* | ATCC 33591 | Susceptible | 1 |
| | ATCC 35556 | Susceptible | 1 |
| | CCARM 3516 | Susceptible | 2 |
| | CCARM 3795 | Susceptible | 0.5 |
| *Staphylococcus epidermidis* | KCTC 3752 | Susceptible | 1 |
| *Staphylococcus haemolyticus* | ATCC 29970 | Susceptible | 1 |
| *Acinetobacter baumannii* | CCARM 12226 | Insusceptible | - |
| *Clostridioides difficile* | CCARM 0185 | Insusceptible | - |
| *Pseudomonas aeruginosa* | CCARM 2252 | Insusceptible | - |
| *Klebsiella pneumoniae* | CCARM 10263 | Insusceptible | - |
| *Escherichia coli* | CCARM 1G937 | Insusceptible | - |

From these results, it was confirmed that the antibacterial activity of the antibacterial protein SSL200 of the present disclosure was significantly specific for *Staphylococci,* including *Staphylococcus aureus.*

From the results, it was confirmed that the antibacterial protein SSL200 specific for *Staphylococci,* including *Staphylococcus aureus* of the present disclosure could lyse *Staphylococci,* including *Staphylococcus aureus,* thereby ultimately contributing to killing *Staphylococci,* including *Staphylococcus aureus.* These antibacterial properties of the antibacterial protein SSL200 showed that the composition containing the antibacterial protein SSL200 specific for *Staphylococci,* including *Staphylococcus aureus* could be used for the purpose of killing *Staphylococci,* including *Staphylococcus aureus* when infections caused by *Staphylococci* occurred. In addition, the antibacterial properties of the antibacterial protein SSL200 showed that the composition could also be used in the same way as conventional antibiotics for the purpose of treating infections caused by *Staphylococci,* including *Staphylococcus aureus.*

### Example 4: Investigation of Antibacterial Activity in Milk of Antibacterial Protein SSL200

The antibacterial activity in milk of the antibacterial protein SSL200 specific for *Staphylococci,* including *Staphylococcus aureus,* and prepared according to Example 1 was examined. Milk was inoculated with *Staphylococcus aureus* ATCC 33591 at a concentration of 3×10⁵ CFU/mL. The SSL200 protein solution was then added thereto to achieve a final concentration of 50 µg/mL. Next, the mixture was left at room temperature for 30 minutes or 1 hour. 50 µL of a sample was obtained from the mixture and then diluted 100-fold. 100 µL of the diluted sample was spread onto agar plates. Thereafter, the plates were incubated for one day. A bacterial count in the milk was measured by using a conventional plate counting method, which was used to measure the number of colonies formed. For the control group, milk was inoculated with *Staphylococcus aureus* ATCC 33591 in the same way, and then a buffer (10 mM L-Histidine, 5% (w/v) Sorbitol, 0.1% (w/ v) poloxamer 188, and pH 6.0) without the SSL200 protein was added to the milk instead of the SSL200 protein solution. Afterward, sample collection, spreading, and culture procedures were performed in the same manner as in the treatment group to which the SSL200 protein solution was added, and then the number of the resulting colonies formed was counted. The results are shown in FIG. 3.

**[Table 3]**

| Results of Bacterial Count Investigation in Milk | | |
|---|---|---|
| Division | Number of Bacteria in 100 µL of Sample | |
| | 30-minute Treatment | 1-Hour Treatment |
| Control Group | 325 | 421 |
| Treatment Group | 4 | 0 |

The results showed that the antibacterial protein SSL200 could exert antibacterial activity in the milk environment. These results showed that the antibacterial protein SSL200 could be used as an active ingredient in treating bovine mastitis.

### Example 5: Investigation of Treatment Effects on Bovine Mastitis

Therapeutic effects of the antibacterial protein SSL200 specific for *Staphylococci,* including *Staphylococcus aureus* on bovine mastitis were investigated. Specifically, whether a composition containing the antibacterial protein SSL200 prepared in Example 2 of the present disclosure was effective in treating bovine mastitis was investigated on 10 dairy cows with bovine mastitis caused by *Staphylococcus aureus.* The dairy cows were divided into two groups of 5 each. One group was administered a composition containing the antibacterial protein SSL200 daily through the nipple. The other group was administered the same composition without the antibacterial protein SSL200 daily through the nipple. The injection volume was 5 mL per time. While this treatment was performed for 10 days, the number of somatic cells contained in the raw milk collected from the dairy cows before and after the 10-day treatment was tested according to a conventional method. When bovine mastitis occurred, white blood cells increased to prevent the invasion of pathogens. At this time, the white blood cells that died through the fight against pathogens were called somatic cells. Types of the somatic cells included mammary epithelial cells, immune cells (white blood cells), neutrophils, lymphocytes, and monocytes. In the experiment, direct microscopy, which was the easiest to perform, was used as a somatic cell examination method. To explain this briefly, raw milk samples were spread, dried, and stained within 1 cm² of a slide glass. Thereafter, somatic cell counts were examined directly under a microscope and multiplied by the microscope count. In this way, the number of somatic cells in 1 mL of raw milk was calculated. The results are as follows. The results in Table 4 below are the average values of 5 animals in each experimental group. Since the values do not deviate significantly from the average, the provision of standard deviation is omitted.

**[Table 4]**

| Treatment Effects of Bovine Mastitis | | |
|---|---|---|
| Division | Somatic Cell Count per mL of Raw Milk (CFU/mL) | |
| | Treatment Group | Control Group |
| Before Treatment | 5.1× 10⁵ | 4.9× 10⁵ |
| After Treatment | 1.7× 10⁴ | 6.7× 10⁵ |

In the table above, the contents corresponding to the treatment group are the results from the group administered the composition containing the antibacterial protein SSL200, meanwhile, the contents corresponding to the control group are the results from the group administered the composition, which did not contain the antibacterial protein SSL200 and had the same remaining composition.

As can be seen from the results, it was confirmed that there was a significant bovine mastitis treatment effect only when the composition containing the antibacterial protein SSL200 of the present disclosure was administered. From these results, it was confirmed that the composition containing the antibacterial protein SSL200 of the present disclosure as an active ingredient was effective in treating bovine mastitis. These properties show that the composition containing the antibacterial protein SSL200 of the present disclosure as an active ingredient could be used for the purpose of treating bovine mastitis.

The specific parts of the present disclosure have been described in detail above. For those skilled in the art, these specific techniques are merely examples of preferred implementations. It is clear that the scope of the present disclosure is not limited by these examples. Accordingly, the actual scope of the present disclosure will be defined by the appended claims and their equivalents.
Depository Institution Name: KCTC
Accession Number: KCTC15030BP
Deposit Date: 20220712

### BUDAPEST TREATY ON THE INTERNATIONAL RECOGNITION OF THE DEPOSIT OF MICROORGANISMS FOR THE PURPOSE OF PATENT PROCEDURE INTERNATIONAL FORM

### RECEIPT IN THE CASE OF AN ORIGINAL DEPOSIT

### Issued pursuant to Rule 7.1

To: iNtRON Biotechnology
Address: 137, Sagimakgol-ro, Jungwon-gu, Seongnam-si, Gyeonggi-do, Republic of Korea

| I. IDENTIFICATION OF THE MICROORGANISM | |
|---|---|
| Identification reference given by the DEPOSITOR: | Accession number given by the INTERNATIONAL DEPOSITARY AUTHORITY: |
| ***Escherichia coli* BM-2** | |
| | **KCTC 15030BP** |

| II. SCIENTIFIC DESCRIPTION AND/OR PROPOSED TAXONOMIC DESIGNATION | |
|---|---|
| The microorganism identified under I above was accompanied by: | |
| [ ] a scientific description | |
| [ ] a proposed taxonomic designation | |
| (Mark with a cross where applicable) | |

| III. RECEIPT AND ACCEPTANCE | |
|---|---|
| This International Depositary Authority accepts the microorganism identified under I above, which was received by it on **July 12, 2022.** | |

| IV. RECEIPT OF REQUEST FOR CONVERSION | |
|---|---|
| This microorganism identified under I above was received by the International Depositary Authority on and a request to convert the original deposit to a deposit under the Budapest Treaty was received by it on. | |

| V. INTERNATIONAL DEPOSITARY AUTHORITY | |
|---|---|
| Name: Korean Collection for Type Cultures | Signature(s) of person(s) having the power to represent the International Depositary Authority or of authorized official(s): |
| Address: 181, Ipsin-gil, Jeongeup-si, Jeonbuk-do, Republic of Korea (56212) | |
| | Representative |
| | Date: **July 12, 2022** |

## Claims

1. An antibacterial protein SSL200 with an amino acid sequence represented by SEQ ID NO: 1, which has a specific antibacterial activity against *Staphylococci,* including *Staphylococcus aureus.*

2. The antibacterial protein SSL200 of Claim 1, wherein the antibacterial protein SSL200 is made of 325 amino acids and has a molecular weight of 35.9 kDa.

3. A composition for treating infections caused by *Staphylococci,* comprising *Staphylococcus aureus,* the composition comprising the antibacterial protein SSL200 of Claim 1 as an active ingredient.

4. The composition of Claim 3, wherein the composition is used as a treatment for bovine mastitis.

5. A method of preparing the antibacterial protein SSL200 of claim 1 by using a production strain *Escherichia coli* BM-2 (accession number KCTC15030BP) constructed by transforming *E. coli* with a plasmid containing a nucleic acid sequence represented by SEQ ID NO: 2.
